# EUROPEAN PATENT SPECIFICATION

(11) **EP 2 632 355 B1**
(45) Date of publication and mention of the grant of the patent: **03.06.2015**
(21) Application number: 11837072.5
(22) Date of filing: 27.10.2011
(51) Int. Cl.: A61B 17/70, A61B 17/86, A61B 17/84, A61F 2/44, A61B 17/88, A61B 19/00

(54) **LOW PROFILE EXTENSION ATTACHMENTS FOR BONE ANCHORS**
NIEDRIGPROFIL-VERSTÄRKUNGSBEFESTIGUNGEN FÜR KNOCHENANKER
FIXATIONS D'EXTENSION À PROFIL BAS POUR DISPOSITIFS D'ANCRAGE OSSEUX

(30) Priority: 27.10.2010 US 913371
(43) Date of publication of application: 04.09.2013
(73) Proprietor: Warsaw Orthopedic, Inc., Warsaw, IN 46581 (US)
(72) Inventor: JUSTIS, Jeff, R., Germantown Tennessee 38139 (US); KAVE, Douglas, D., Byhalia Mississippi 38611 (US)
(74) Representative: Viering, Jentschura & Partner Patent- und Rechtsanwälte
(86) International application number: PCT/US2011/058040
(87) International publication number: WO 2012/058402

(56) References cited:
- WO-A1-2009/132110
- US-A1- 2007 049 931
- US-A1- 2007 191 840
- US-A1- 2008 082 103
- US-A1- 2008 091 213
- US-A1- 2009 228 052
- US-B2- 7 491 208

## Description

### BACKGROUND

Various devices and methods for stabilizing bone structures have been used for many years. For example, the fracture of an elongated bone, such as a femur or humerus, can be stabilized by securing a plate to the fractured bone across the fracture. The plate extends across the fractured area and thus stabilizes the fractured components of the bones relative to one another in a desired position. When the fracture heals, the plate can be removed or left in place, depending on the type of plate that is used.

Another type of stabilization technique uses one or more elongated rods extending between components of a bony structure and secured to the bony structure to stabilize the components relative to one another. The components of the bony structure are exposed and one or more bone engaging fasteners are placed into each component. The elongated rod is then secured to the bone engaging fasteners in order to stabilize the components of the bony structure.

One problem associated with the above described stabilization structures is that the skin and tissue surrounding the surgical site must be cut, removed, and/or repositioned in order for the surgeon to access the location where the stabilization device is to be installed. This repositioning of tissue causes trauma, damage, and scarring to the tissue. There are also risks that the tissue will become infected and that a long recovery time will be required after surgery for the tissue to heal.

Minimally invasive surgical techniques are particularly desirable in, for example, spinal and neurosurgical applications because of the need for access to locations deep within the body and the presence of vital intervening tissues. The development of percutaneous minimally invasive spinal procedures has yielded a major improvement in reducing recovery time and post-operative pain because they require minimal, if any, muscle dissection and can be performed under local anesthesia. These benefits of minimally invasive techniques have also found application in surgeries for other locations in the body where it is desirable to minimize tissue disruption and trauma. However, there remains a need for further improvements in instruments, systems and methods for stabilizing bony structures using minimally invasive techniques.

A spinal surgical system is known from US 2009/228052 A1, from which the preamble of claim 1 derives.

### SUMMARY

The invention provides a system according to claim 1 for positioning a connecting member adjacent the spinal column that includes at least one anchor assembly having an anchor engageable to bony structure and a receiver for receiving the connecting member. An extension is removably engaged to the receiver and extends proximally from the receiver to a proximal end located outside the patient. The extension and at least a portion of the receiver are removable from the remaining portion of the receiver of the bone anchor after the connecting member is positioned in the receiver to provide a low profile anchor assembly when the connecting member and bone anchor are finally implanted in the patient. Further embodiments of the invention are described in the dependent claims.

These and other aspects will be apparent from the following description of the illustrated embodiments.

### BRIEF DESCRIPTION OF THE FIGURES

Fig. 1 is a perspective view of an anchor assembly with an extension engaged to an anchor and a connecting member positioned through the anchor.
Fig. 2 is an exploded perspective view of the anchor assembly of Fig. 1 and an engaging member engageable to the anchor.
Fig. 3 is a perspective view of first and second extension members of the extension of Fig. 1.
Fig. 3A is a section view along line 3A-3A of Fig. 3.
Fig. 4 is a longitudinal section view of the anchor assembly of Fig. 1 showing various locations for the connecting member relative thereto.
Fig. 5 is a perspective view of the receiver of the anchor assembly of Fig. 1.
Fig. 6 is an exploded perspective view of the bone anchor portion of the bone anchor assembly of Fig. 1 that is engageable with the receiver of Fig. 5.
Fig. 7 is a diagrammatic perspective view of a spinal column segment having multiple anchor assemblies and extensions engaged thereto and a connecting member inserter.
Fig. 8 is another diagrammatic perspective view of the spinal column segment and anchor assemblies and extensions of Fig. 7 with at least one of the anchor assemblies and extensions manipulated to align the vertebrae and mount to the connecting member inserter.

### DESCRIPTION OF THE ILLUSTRATED EMBODIMENTS

For the purposes of promoting an understanding of the principles of the invention, reference will now be made to the embodiments illustrated in the drawings and specific language will be used to describe the same. It will nevertheless be understood that no limitation of the scope of the invention is thereby intended.

Anchor assemblies are provided that are engageable to a bony structure, such as one or more vertebrae of a spinal column, to guide placement of one or more connecting members from a location outside patient, or within the patient but remote from an implantation location, to or more adjacent to an implantation location in the patient. The connecting member can be an elongated spinal rod, tether, bar, plate, wire, or other suitable device that is to be engaged to one or more bone anchors. The anchor assemblies are particularly suited for minimally invasive surgical procedures, but are not restricted to such procedures. Furthermore, although its use and application is described with regard to spinal surgery, applications in surgeries other than spinal surgery are also contemplated.

In one form, surgical systems are provided that include at least one anchor assembly engageable to a spinal column. The anchor assembly includes a bone anchor with a bone engaging portion and a receiver for receiving the connecting member. An elongated extension is engaged to and extends proximally from the receiver to a proximal end located so that it is readily accessible by the surgeon. The extension includes first and second elongated extension members that define a space between the elongate members that extends proximally from a proximal end opening of the receiver located between the elongate members. The elongate members can provide a path for insertion of the connecting member that is referenced to the location of the receiver, or a platform for engagement of an inserter to proximal ends of the elongate members so that the inserter is references to the receiver locations within the patient. The connecting member is positionable into the space defined by the elongate members and movable along the extension through a proximal end opening of the receiver and into the receiver. The connecting member can also be positioned directly into the receiver from a side of the receiver. The connecting member can then be secured to the bone anchor with an engaging member engaged to the receiver. The elongate members of the extension are removable from the receiver so that the anchor assembly has a low-profile in the patient post-surgery. In one embodiment, a proximal portion of the receiver member is separated from a distal portion of the receiver to remove the elongate member mounted to the proximal portion of the receiver.

Further, surgical systems are provided that include at least a pair of anchor assemblies engaged to the spinal column. The anchor assemblies each include an extension engaged to a receiver of the bone anchor so that the extension extends proximally from the receiver. The anchor assemblies are implanted into the patient while the extensions extend proximally away from the implantation location. The extensions provide a platform for engagement of an inserter, or can serve as a guide for the placement of a connecting member from a position remote from implantation location to a position more adjacent the spinal column, such as the implantation location. The extensions are configured so that when the connecting member is guided adjacent to the spinal column, the connecting member extends through the bone anchors of the anchor assemblies. The connecting member is secured to the bone anchor assemblies and provides stabilization of the spinal column segment to which the bone anchors assemblies are attached. The extensions are removed from the bone anchor assemblies after implantation of the connecting member so that the connecting member implantation and extension removal is accomplished without invasively accessing the patient's body. In one embodiment, a portion of the receiver member to which the extension is mounted is separated from the remaining portion of the receiver in order to remove a corresponding member of the extension engaged thereto. In a further embodiment, a connecting member inserter is mounted to the proximal end of one or more the extensions to reference the connecting member to the anchor assembly locations and to guide placement of the connecting member to the bone anchor assemblies in the patient.

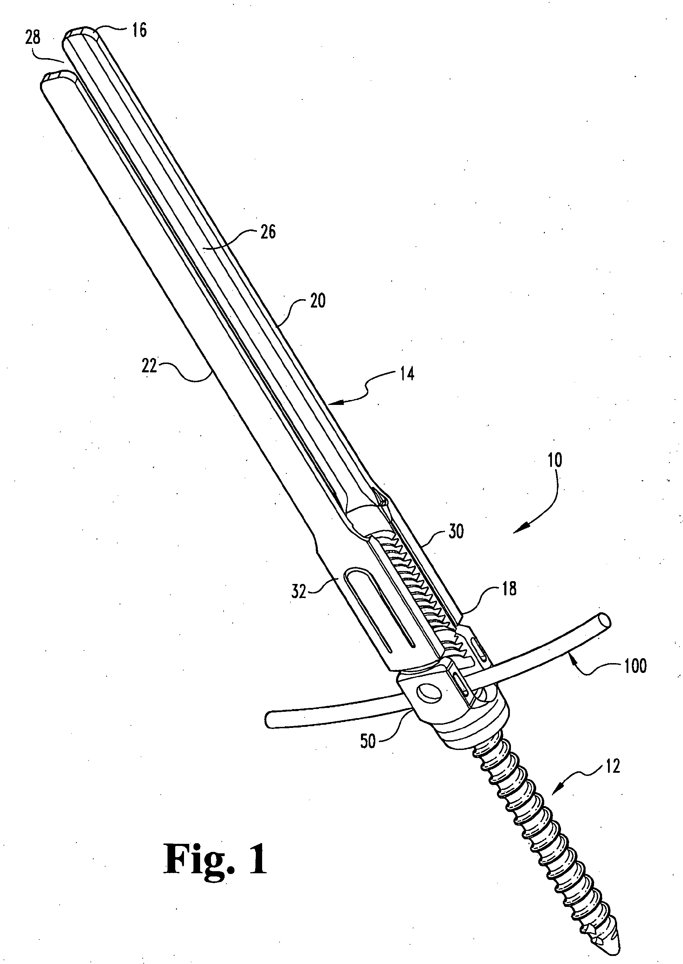

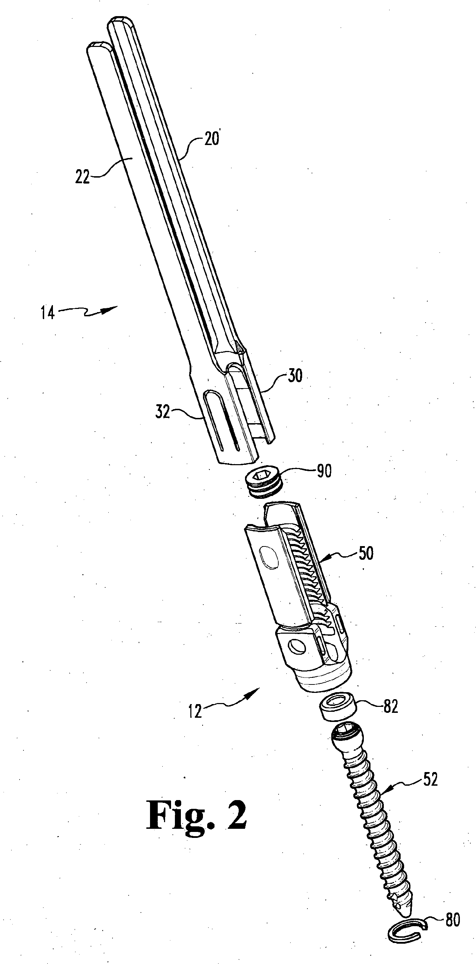

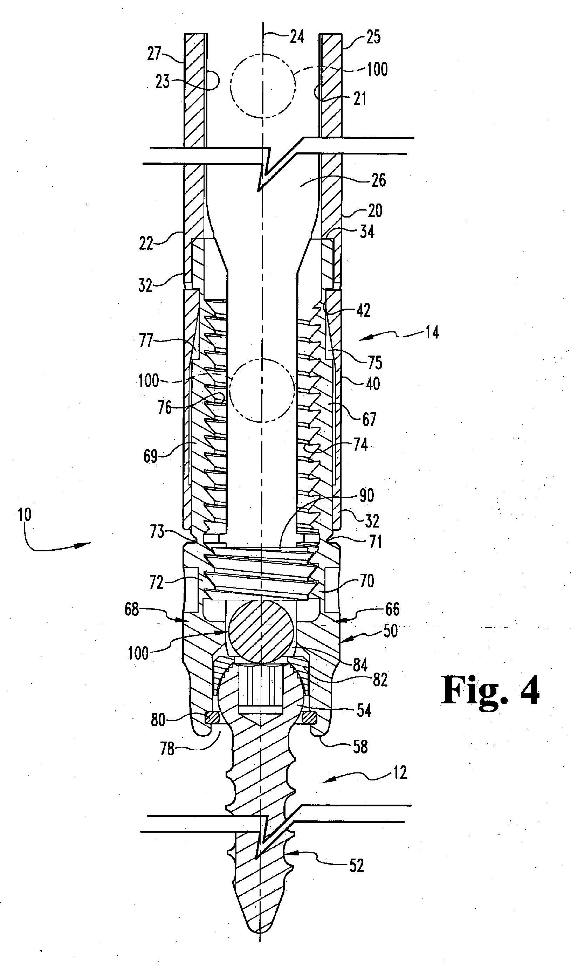

Referring to Figs. 1-2, one embodiment of an anchor assembly 10 is shown with an extension 14 mounted thereto. Anchor assembly 10 includes a bone anchor 12 and an extension 14 extending proximally from bone anchor 12 to a proximal end 16. Extension 14 extends from proximal end 16 to a distal end 18 where it is secured to bone anchor 12 so that the bone anchor and extension form an assembly that is implantable as a unit into the patient. Extension 14 includes elongate first and second extension members 20, 22 extending longitudinally on opposite sides of a central longitudinal axis 24 (Fig. 4) from distal end 18 to proximal end 16. Elongate members 20, 22 form a space 26 therebetween that can receive the connecting member 100 between members 20, 22.

In one embodiment, elongate members 20, 22 include a length sufficient to locate proximal end 16 outside the skin and tissue of the patient when bone anchor 12 is secured to bony structure within the patient. In the illustrated embodiment, elongate members 20, 22 form a proximal opening 28 therebetween to allow the connecting member to be placed through the proximal end opening 28 into space 26 between elongate members 20, 22. In an alternative embodiment, a proximal portion can be provided that extends between and connects elongate members 20, 22 to close the proximal end opening, requiring connecting member 100 to be positioned into space 26 from the side opening between members arms 20, 22 with connecting member 100 oriented in a length-wise manner that is transverse to longitudinal axis 24.

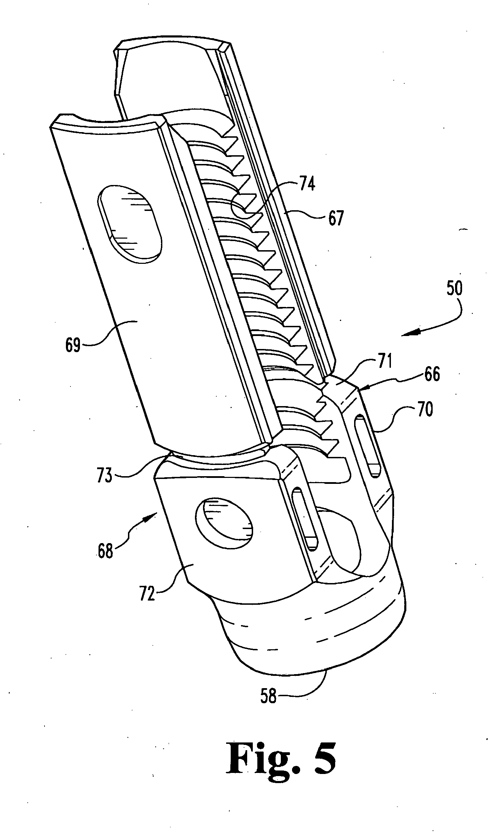

Referring further to Figs. 4 and 5, bone anchor 12 includes a proximal receiver 50 and a distal bone engaging portion 52. Each of the elongate members 20, 22 is positioned on receiver 50 so that anchor assembly 10 is provided, at least initially to the surgeon, as a unit that includes anchor 12 and extension 14 for simultaneous implantation into the patient. Elongate members 20, 22 are removably engaged to receiver 50 so that elongate members 20, 22 and bone anchor 12 can be initially provided in a separated condition and then assembled prior to surgery. This allows the surgeon to select the optimal extension length and bone anchor combination to employ during the surgical procedure. In addition, elongate extension members 20, 22 can be re-used in subsequent procedures since the removal of extension 14 from receiver 50 involves separating a portion of receiver 50 while the elongate members 20, 22 remain intact. Furthermore, the separated portion of receiver 50 remains engaged to the corresponding elongate member 20, 22 mounted thereto so that separated receiver portion is removed from the patient when the corresponding elongate member 20, 22 is withdrawn from the patient. When extension 14 is removed from bone anchor 12, bone anchor 12 is configured for post-operative implantation in the patient with connecting member 100 in receiver 50.

In the illustrated embodiment, receiver 50 forms a saddle that houses a portion of bone engaging member 52 and connecting member 100. Receiver 50 receives the connecting member 100 therethrough in an orthogonal or transverse orientation to longitudinal axis 24 and in an orientation that extends generally parallel with the spinal column. In one embodiment, connecting member 100 is an elongated spinal rod, and bone anchor 12 includes a bone screw portion extending from a distally facing end of receiver 50. The bone screw portion can be a multi-axial type screw pivotally received and carried by receiver 50 so that the receiver and bone screw are pivotal relative to one another. In another embodiment, the bone screw portion is non-pivotal or fixed relative to the receiver. Furthermore, connecting member 100 can be received in, on, or about receiver 50 for engagement thereto with an engaging member 90. Engaging member 90 is shown in Fig. 4 as an externally threaded set screw. However, other embodiments contemplate engaging members that include one or more components in the form of a nut, cap, non-threaded member, friction fit member, twist-lock member, or combinations thereof that engage the receiver. Furthermore, connecting member 100 can be rigid, semi-rigid, flexible, elastic, non-compression load bearing, or of other suitable form for extending between and stabilizing adjacent portions of the spinal column when secured thereto with one or more bone anchors.

In the illustrated embodiment, receiver 50 includes a pair of opposite side arms 66, 68 sized and spaced to accommodate connecting member 100 and engaging member 90 therebetween. Arms 66, 68 each include a removable break-off portion 67, 69 extending to a proximal end of receiver 50. Elongate members 20, 22 of extension 14 form an elongated extension of respective ones of the side arms 66, 68, and are mounted thereto along the outer surface of the respective break-off portion 67, 69. Break-off portions 67, 69 are joined to a respective distal portion 70, 72 of arms 66, 68 at a separation location formed by a reduced thickness in the respective arm 66, 68 at break-off recess 71, 73, respectively. Recesses 71, 73 extend into and form an indentation in the outer surfaces of arms 66, 68 and a groove in the inner surfaces of arms 66, 68. Break-off recesses 71, 73 provide an area of reduced wall thickness along arms 66, 68 so that the break-off portions 67, 69 can be removed by application of a threshold torque and/or shear force to the respective elongate member 20, 22 mounted thereto. When the break-off portion 67, 69 is separated, it is removed by withdrawing the respective elongate member 20, 22 from the patient since, as discussed further below, the break-off portion 67, 69 is mounted to the elongate member 20, 22. In one embodiment, recesses 71, 73 extend completely about the respective arm 66, 68, including its outer and inner surfaces, and interrupt the inner thread profiles 74, 76 thereof. Further examples of suitable receivers are disclosed in U.S. Patent App. Pub. No. 2007/0191840.

Elongate members 20, 22 each include a length extending proximally from break-off portions 67, 69 so that the proximal ends of elongate members 20, 22 are located outside the patient when anchor 12 is engaged to the spinal column. In one embodiment, this length is at least 30 millimeters. In another embodiment, the length of extension members 20, 22 is at least 50 millimeters. Other lengths are also contemplated. In one specific embodiment, the length is at least 100 millimeters, and extends about 120 millimeters from the anchor 12 to the proximal end 16 of extension 14.

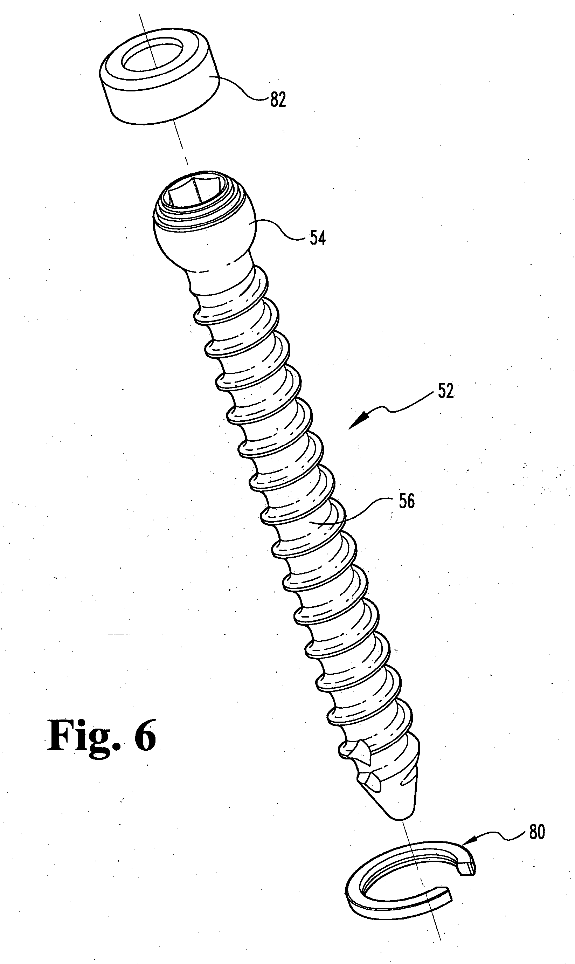

Arms 66, 68 each include an internal thread profile 74, 76 that extends along the inner surfaces of the break-off portions 67, 69 and distal portions 70, 72 of arms 66, 68 to threadingly receive and engage engaging member 90. In other embodiments, break-off portions 67, 69 do not include any thread profile. Receiver 50 includes a hole 78 extending on longitudinal axis 24 that opens through a distally facing surface 58 of receiver 50. Hole 78 is sized and shaped to receive bone engaging member 52 therethrough while supporting head 54 of bone engaging member 52 in receiver 50. Near the distally facing surface 58 at the bottom of receiver 50, hole 78 is surrounded by a retaining member 80, as is further shown in Fig. 6. Retaining member 80 can be C-ring, washer, lip, or flange formed separately from or as an integral part of receiver 50 to support head 54 while allowing bone engaging member 52 to be positioned in any one of an infinite number of angular positions relative to receiver 50 and longitudinal axis 24. Other embodiments contemplate other engagement relationships between the bone engaging member 52 and receiver 50. In one embodiment, bone engaging member 52 is formed as a single, integral unit with receiver 50 and extends along longitudinal axis 24 in a co-axial arrangement. In another embodiment, bone engaging member 52 is captured in receiver 50 in a manner that allows pivotal movement relative to receiver 50 in a single plane or in a predetermined number of planes or directions relative to longitudinal axis 24.

In the particular illustrated embodiment of bone engaging member 52, it includes an initial configuration that allows pivoting movement in receiver 50 and is thereafter rigidly or semi-rigidly fixed in position when connecting member 100 is seated in receiver 50. Receiver 50 includes a crown 82, also shown in Fig. 6, positioned on and around the proximal side of head 54. Crown 82 includes a proximal side that projects into a passage 84 defined between side portions 66, 68. When engaging member 90 is threadingly engaged to receiver 50, it pushes connecting member 100 in passage 84 against the proximal side of crown 82. Crown 82 is in turn pushed against the proximal side of head 54, which seats head 54 firmly against retaining member 80. The proximal side of head 54 may include a plurality of ridges or grooves that bite into a distally facing surface of crown 82 to enhance locking of bone engaging member 52 in position in receiver 50. In another embodiment, at least some motion between the receiver 50 and bone engaging member 52 is maintained by crown 82 when connecting member 100 is secured in receiver 50 with engaging member 90. Still other embodiments contemplate that crown 82 can be omitted and that connecting member 100 is seated directly against head 54 of bone engaging member 52 or against a bottom surface 55 of receiver 50 that extends on a distal side of passage 84 between side portions 66, 68.

Bone engaging portion 52 is shown as a bone screw with proximal head 54 and an elongated threaded shaft 56 extending distally from head 54 located in receiver 50. Other embodiments contemplate other forms for bone engaging member 52, such as a hook, post, tack, cerclage, staple, anchor, or other suitable bone engaging structure. Bone engaging member 52 can be a separate member that is connected with receiver 50, or formed as an integral, one-piece construct with receiver 50.

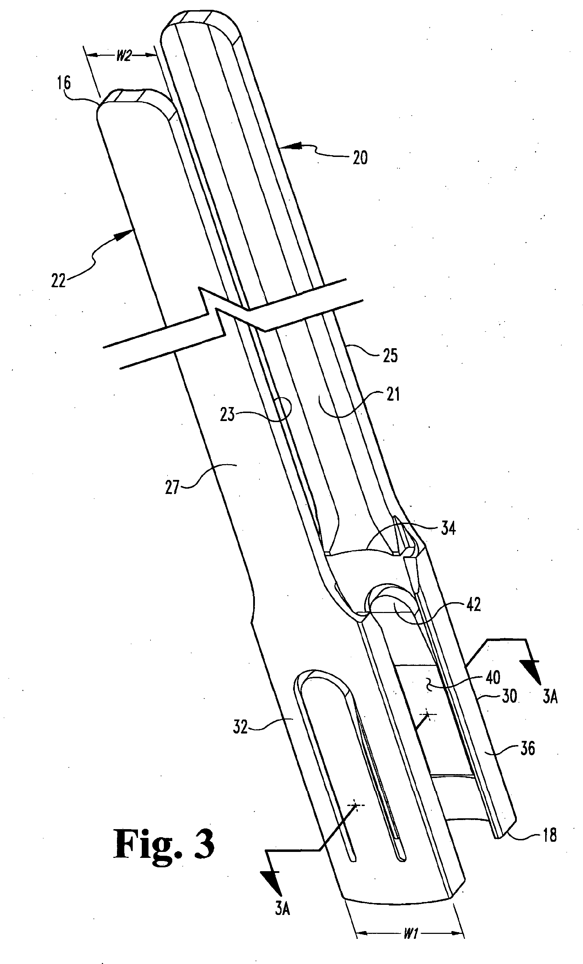

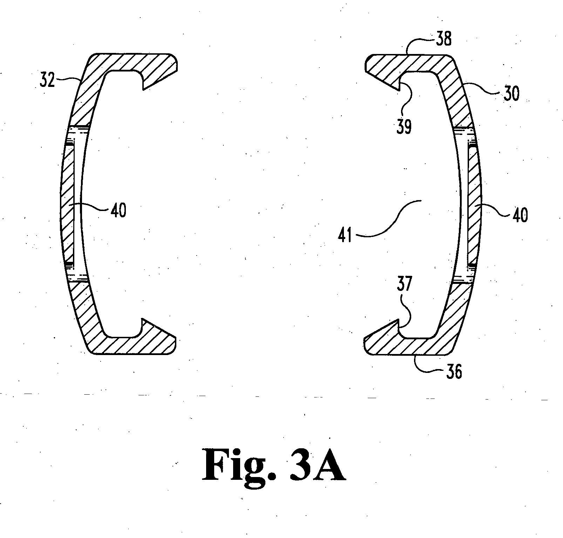

Referring further to Fig. 3, extension 14 includes elongate members 20, 22 extending parallel or substantially parallel to one another distally from proximal end 16 to distal end 18. Elongate members 20, 22 each include a distal mounting structure 30, 32 that receives and is mounted to the corresponding break-off portion 67, 69 of receiver 50. Members 20, 22 also include proximal portions that extend proximally from mounting structures 30, 32 to proximal end 16. The proximal portions include planar or generally planar facing surfaces 21, 23 extending parallel to one another and parallel to longitudinal axis 24 that define space 26 therebetween. In addition, elongate members 20, 22 include outer, oppositely facing surfaces 25, 27, respectively, that define any one or combination of a planar surface, a convex curvature, a concave curvature extending around longitudinal axis 24. Outer facing surfaces 25, 27 define a generally linear profile paralleling longitudinal axis 24. In the illustrated embodiment, the outer surfaces 25, 27 provide a smooth surface contour that holds back tissue from encroaching into space 26 while minimizing trauma to the tissue pressing against elongate members 20, 22.

Outer surfaces 25, 27 extend generally parallel to longitudinal axis 24 from proximal end 16 to distal end 18. Elongate members 20, 22 are substantially identical to one another, so the description of mounting structure 30 of elongate member 20 applies to mounting structure 32 of elongate member 22. However, other embodiments contemplate embodiments in which elongate members 20, 22 include differing configurations and/or mounting structures. Elongate members 20, 22 include a width that increases along mounting structures 30, 32 to accommodate placement over the respective break-off portion 67, 69. Mounting structures 30, 32 include a lip 34 projecting inwardly therefrom that forms a distally facing surface that contacts the proximal end of the respective break-off portion 67, 69 to limit distal advancement of the elongate member 20, 22 along the respective break-off portion 67, 69. The reduced width proximal portion of members 20, 22 extending proximally from the respective mounting structure 30, 32 provides a low profile footprint that minimizes intrusion into the adjacent tissue. Thus, mounting structures 30, 32 can include a width W1 that is more than 0 percent and up to about 50% larger than width W2 of the proximal portion of member 20, 22. However, embodiments in which width W2 is the same or greater than width W1 are not precluded.

Referring further to Fig. 3A, mounting structures 30, 32 also include side rails 36, 38 on opposite sides thereof that receive and bracket the sides of the respective break-off portion 67, 69. Side rails 36, 38 include a flange 37, 39 that extends along the inner side of the break-off portion 67, 69 to provide a secure connection of mounting structure 30, 32 to the break-off portion 67, 69. Side rails 36, 38 define a channel 41 along the inner side of the mounting structure 30, 32 that slidingly receives the break-off portion 67, 69 that minimizes or eliminates play or movement therebetween, providing an intimate fit and secure frictional connection with the break-off portion 67, 69. In addition, mounting structure 30, 32 includes a spring loaded tab 40 with a proximal protrusion 42 that extends inwardly from the inner facing surface of mounting structure 30, 32. Protrusion 42 is received in a recess 75, 77 formed in the outer surface of the respective break-off portion 67, 69. Protrusion 42 forms a proximally facing surface that, along with lip 34, maintains the distal-proximal securing location of elongate member 20, 22 along the break-off portion 67, 69. The interface of mounting structure 30, 32 with break-off portion 67, 69 holds the break-off portion 67, 69 in mounting structure 30, 32 when the break-off portion is separated so it can be removed from the patient by withdrawing the member 20, 22 mounted thereto. In the illustrated embodiment, tab 40 includes a distal end that is formed as a living or integral hinge with elongate member 20, 22, and extends proximally from the hinge to its proximal end where protrusion 42 is located. Material is removed around the remaining three sides of tab 40 so that it can flex or bend inwardly and outwardly relative to mounting structure 30, 32.

Extension 14 also includes features to facilitate guiding and placement of connecting member 100 into passage 84 of receiver 50. Elongate members 20, 22 include a receiving portion along inner surfaces 21, 23 that can form a location to receive and provide initial guidance of the placement of connecting member 100 into and along space 26. Elongate members 20, 22 also direct connecting member 100 from its initial placement through space 26 into alignment with the space between arms 66, 68 and passage 84. As discussed further below, if connecting member 100 is not entirely located in passage 84, engaging member 90 can be threaded along the inner thread profiles 74, 76 of arms 66, 68 to contact and displace connecting member 100 into passage 84. Providing arms 66, 68 with threaded break-off portions 67, 69 provides a greater range of locations along the arms 66, 68 of receiver 50 through which connecting member 100 can be received and then reduced into passage 84 with engaging member 90.

As shown in Fig. 4, connecting member 100 can be initially positioned through space 26 of extension 14 at a location between inner surfaces 21, 23 at or near the proximal end 16 of extension 14. Alternatively, connecting member 100 can initially be positioned in the space between break-off portions 67, 69, or directly into passage 84 between distal portions 70, 72 of arms 66, 68. If necessary, connecting member 100 is advanced distally in extension 14 toward receiver 50 into passage 84 by a reducing instrument or by threadingly advancing engaging member 90 along arms 66, 68. Inner surfaces 21, 23 of members 20, 22 and/or the inner surfaces of arms 66, 68 center connecting member 100 in extension 14 and align it with passage 84 of receiver 50.

In use, extension 14 is positioned on receiver 50 so that anchor assembly 10 is provided, at least initially to the surgeon, as a unit that includes anchor 12 and extension 14 for simultaneous implantation into the patient. In one embodiment, extension 14 is made from a radiolucent material so that radiographic or fluoroscopic visualization of connecting member 100 is not obscured between members 20, 22, allowing the surgeon to monitor advancement of connecting member 100 along extension 14 and through the tissue of the patient during the procedure. Examples of suitable materials for extension 14 include polyetheretherketone (PEEK), plastics, polymers, or aluminum, for example. Other materials are also contemplated, including radio-opaque materials. Still other embodiments contemplate that extension 14 is made from a non-conductive material so that probes, taps, drivers and other instruments that employ electrical signals for neuro-stimulation are not shunted through extension 14 to the tissue around extension 14. Extension 14 may also include one or more holes, rockers, notches, or other feature to allow attachment of an inserter or a surgical reduction instrument to mechanically facilitate placement of connecting member 100 into passage 84 of receiver 50 and/or to align a vertebra attached to extension 14.

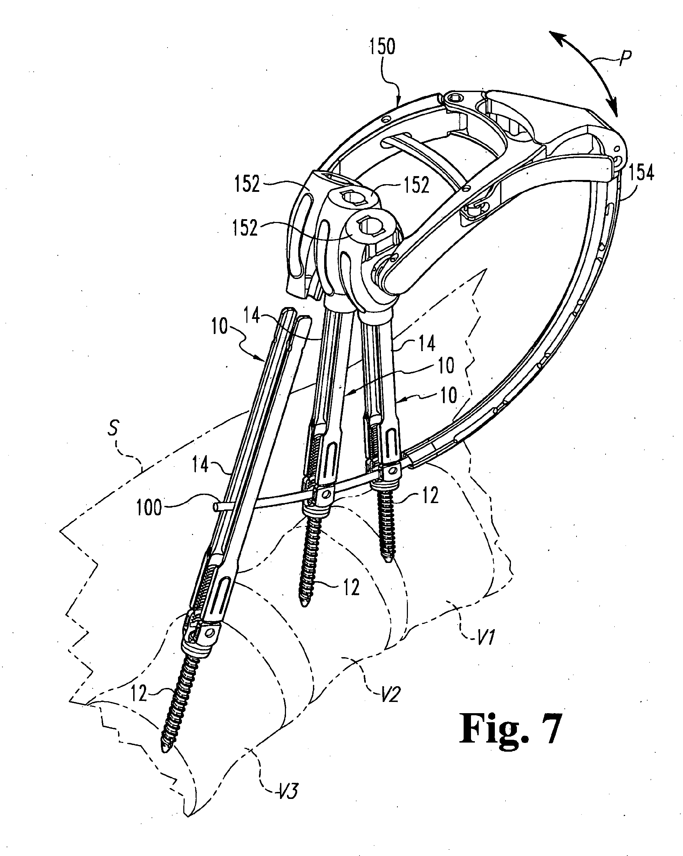

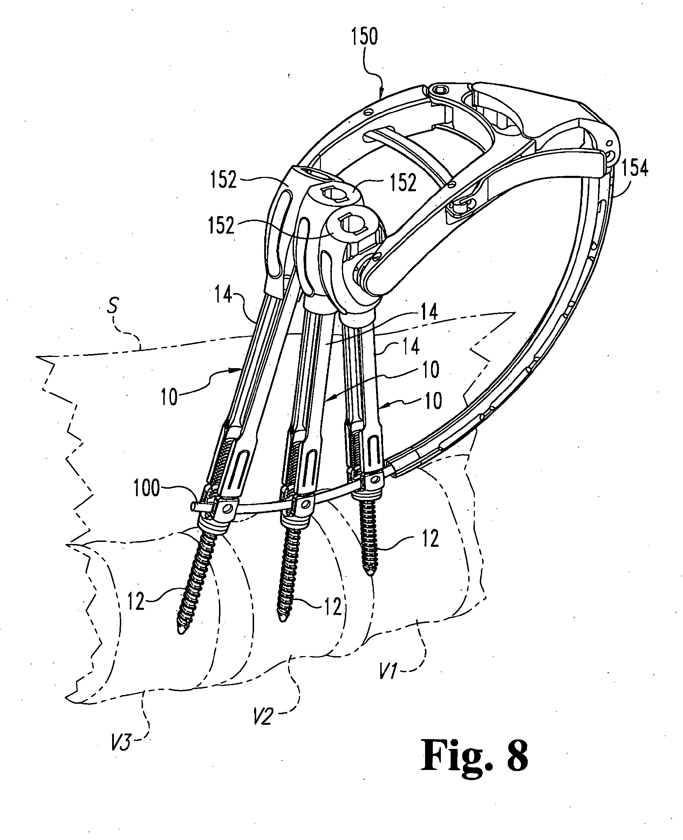

Referring to Figs. 7-8, there is shown an example of a system employing multiple anchor assemblies 10 with bone anchors 12 engaged to respective ones of the vertebrae V1, V2, V3. Although three anchor assemblies 10 and vertebrae are shown, systems employing one, two, or four or more anchor extensions and vertebrae are also contemplated. Extensions 14 extend proximally from respective ones of the bone anchors 12 through the tissue of the patient and skin S to locate the proximal ends of extensions 14 outside the patient. Incisions are made to accommodate insertion of the anchors assemblies 10. Separate incisions can be made for each anchor assembly 10, or a common incision for all anchor assemblies 10. An inserter 150 includes connectors 152 that are mounted to the proximal ends of corresponding ones of the extensions 14 outside the patient. Inserter 150 also includes an inserter arm 154 extending from and pivotally mounted to connectors 152. Connecting member 100 is engaged to the end of inserter arm 154 so that inserter arm 154 can be pivoted relative to extensions 14, as indicated by arrows P, to advance connecting member 100 through the tissue of the patient so that connecting member 100 extends through the anchor assemblies 10. Alternatively, connecting member 100 can be guided axially along one of the extension 14 and then pivoted adjacent to anchors 12 to extend between each of the anchors 12, or positioned to extend transversely to and between two or more of the extensions 14 and then guided down the extensions 14 in the transverse orientation to anchors 12. Extensions 14 also provide a pathway to allow placement of engaging members 90 therealong to secure connecting member 100 to the respective anchor 12. Examples of suitable inserters and connectors for mounting to the proximal ends of extensions 14, and insertion techniques for connecting member 100 may be found in U.S. Patent Nos. 6,530,929; 7,188,626; 7,465,306; 7,520,879; 7,597,694 and in U.S. Patent App. Pub. Nos. 2007/0049931 and 2008/0319477.

Figs. 7-8 also show an example of a technique for reducing connecting member 100 into anchor 12 of one of the anchors assemblies 10. As shown in Fig. 7, vertebra V3 is not aligned with vertebrae V1 and V2, thus the anchor 12 engaged to vertebra V3 is not positioned to receive connecting member 100. Connecting member 100 extends through space 26 of extension 14. The extension 14 mounted to vertebra V3 can be manipulated to pull it and vertebrae V3 proximally for mounting extension 14 in the corresponding connector 152 of inserter 100. When connecting member 100 is aligned with the break-off portions 67, 69 of receiver 50, engaging member 90 can be engaged to the inner thread profile and threadingly advanced into receiver 50 to force connecting member 100 into passage 84 along distal portions 70, 72 of receiver 50. When connecting member 100 is secured to each of the anchors 12, elongate members 20, 22 of extensions 14 can be manipulated by pulling or twisting to separate the respective break-off portions 67, 69 engaged thereto from receiver 50 to allow the break-off portion end elongate member mounted thereto to be removed, providing a modified, low profile anchor assembly for implantation in the patient. In other embodiments, a separate reduction instrument is positioned in or around extension 14 and manipulated to move connecting member 100 into passage 84 along distal portions 70, 72 of receiver 50.

While the invention has been illustrated and described in detail in the drawings and foregoing description, the same is to be considered as illustrative and not restrictive in character.

## Claims

1. A spinal surgical system, comprising:
a connecting member (100) including an elongated body having a length sized to extend between at least two vertebrae (V1, V2, V3); and
an anchor assembly (10) including a distal bone engaging portion (52) engageable to bony structure and a receiver (50) extending proximally from said bone engaging portion (52), said receiver (50) including a passage between opposite arms (66, 68) of said receiver (50) with said passage opening at opposite sides of said arms (66, 68) of said receiver (50) and with said passage further opening at a proximal end of said receiver (50), wherein said arms (66, 68) each include a distal portion (70, 72) along said passage and a proximal break-off portion (67, 69) extending proximally from said distal portion (70, 72), **characterised in that** said anchor assembly (10) further including an extension (14) having a pair of elongated members (20, 22) extending along a longitudinal axis from a proximal end of a respective one of said pair of elongated members (20, 22) to said break-off portions (67, 69) of said receiver (50), each of said elongated members (20, 22) of said extension (14) including a distal mounting structure (30, 32) that captures said break-off portion (67, 69) of said respective arm (66, 68) so that when said break-off portion (67, 69) is separated from said distal portion (70, 72) of said respective arm (66, 68) said elongate member (20, 22) and said break-off portion (67, 69) remain engaged to one another.

2. The system of claim 1, wherein said elongated members (20, 22) of said extension (14) define a space therebetween that extends to said passage of said receiver (50), wherein said space and said passage are sized to receive said connecting member (100) therethrough so that connecting member (100) is movable from said space through said proximal end opening of said passage into said receiver (50).

3. The system of claim 1, wherein said mounting structure (30, 32) includes rails (36, 38) extending along opposite sides thereof that define a channel (41) in which said break-off portion (67, 69) of said respective arm (66, 68) of said receiver (50) is positioned, said mounting structure (30, 32) capturing said break-off portion (67, 69) in said channel thereof.

4. The system of claim 3, wherein said mounting structure (30, 32) includes a tab (40) having a protrusion (42) that releasably engages a recess (75, 77) formed in an outer surface of said break-off portion (67, 69) of said respective arm (66, 68) of said receiver (50).

5. The system of claim 1, wherein said break-off portion (67, 69) is joined to said distal portion (70, 72) by a reduced wall thickness section of said respective arm (66, 68).

6. The system of claim 5, wherein said reduced wall thickness is formed by a groove (71, 73) that extends completely around said arm (66, 68).

7. The system of claim 1, wherein said arms (66, 68) each include an inner thread profile (74, 76) that extends along said break-off portion (67, 69) and said distal portion (70, 72) thereof, and further comprising an engaging member (90) engageable to said inner thread profiles (74, 76) to secure the connecting member (100) in the passage, wherein said break-off portions (67, 69) are joined to said distal portions (70, 72) with a reduced thickness region of said arm (66, 68) that defines a separation location between said distal portion (70, 72) and said break-off portion (67, 69).

8. The system of claim 7, wherein said mounting structure (30,32) includes:
rails (36, 38) extending along opposite sides thereof that define a channel in which said break-off portion (67, 69) of said receiver (50) is positioned; and
a tab (40) having a protrusion (42) that releasably engages a recess (75, 77) formed in said break-off portion (67, 69) of said receiver (50), wherein said tab (40) include a hinged connection with said elongate member (20, 22) at a distal end of said tab (40) and said tab (40) extends proximally from said hinged connection to a proximal end where said protrusion (42) is located.

9. The system of claim 8, wherein said mounting structure (30, 32) includes a lip (34) projecting therefrom that contacts a proximal end of said break-off portion (67, 69) when said protrusion (42) of said tab (40) is received in said recess (75, 77).

10. The system of claim 1, wherein each mounting structure (30, 32) is removably engaged to said break-off portion (67, 69) of a respective arm (66, 68) of said receiver (50).

## Patentansprüche

1. Wirbelsäulen-Operationssystem, aufweisend
ein Verbindungselement (100), das einen länglichen Körper aufweist, welcher eine Länge hat, die so bestimmt ist, dass sie sich zwischen zumindest zwei Wirbeln (V1, V2, V3) erstreckt, und
eine Ankereinrichtung (10) aufweisend einen distalen Knocheneingriffsabschnitt (52), der mit einer knochigen Struktur in Eingriff bringbar ist, und eine Aufnahme (50), die sich proximal von dem Knocheneingriffsabschnitt (52) erstreckt, wobei die Aufnahme (50) eine Passage zwischen gegenüberliegenden Armen (66, 68) der Aufnahme (50) aufweist, wobei die Passage an gegenüberliegenden Seiten der Arme (66, 68) der Aufnahme (50) offen ist, und wobei die Passage ferner an einem proximalen Ende der Aufnahme (50) offen ist, wobei die Arme (66, 68) jeweils einen distalen Abschnitt (70, 72) entlang der Passage und einen proximalen Abbruch-Abschnitt (67, 69), der sich proximal von dem distalen Abschnitt (70, 72) erstreckt, aufweisen, **dadurch gekennzeichnet, dass** die Ankereinrichtung (10) ferner eine Verlängerung (14) aufweist, die ein Paar von länglichen Elementen (20, 22) aufweist, welche sich entlang einer Längsachse von einem proximalen Ende eines jeweiligen des Paares von länglichen Elementen (20, 22) zu den Abbruch-Abschnitten (67, 69) der Aufnahme (50) erstrecken, wobei jedes der länglichen Elemente (20, 22) der Verlängerung (14) eine distale Montagestruktur (30, 32) aufweist, die den Abbruch-Abschnitt (67, 69) des jeweiligen Arms (66, 68) erfasst, so dass, wenn der Abbruch-Abschnitt (67, 69) von dem distalen Abschnitt (70, 72) des jeweiligen Arms (66, 68) getrennt wird, das längliche Element (20, 22) und der Abbruch-Abschnitt (67, 69) miteinander im Eingriff bleiben.

2. System gemäß Anspruch 1, wobei die länglichen Elemente (20, 22) der Verlängerung (14) einen dazwischenliegenden Raum definieren, der sich zu der Passage der Aufnahme (50) erstreckt, wobei der Raum und die Passage eine Größe haben, um das Verbindungselement (100) dort hindurch aufzunehmen, so dass das Verbindungselement (100) von dem Raum durch die proximale Endöffnung der Passage in die Aufnahme (50) hinein bewegbar ist.

3. System gemäß Anspruch 1, wobei die Montagestruktur (30, 32) Schienen aufweist (36, 38), die sich entlang gegenüberliegender Seiten davon erstrecken und die einen Kanal (41) definieren, in welchem der Abbruch-Abschnitt (67, 69) des jeweiligen Arms (66, 68) der Aufnahme (50) positioniert ist, wobei die Montagestruktur (30, 32) den Abbruch-Abschnitt (67, 69) in dem Kanal davon erfasst.

4. System gemäß Anspruch 3, wobei die Montagestruktur (30, 32) eine Lasche (40) aufweist, die einen Vorsprung (42) hat, der lösbar in eine Aussparung (75, 77), die in einer äußeren Fläche des Abbruch-Abschnitts (67, 69) des jeweiligen Arms (66, 68) der Aufnahme (50) gebildet ist, eingreift.

5. System gemäß Anspruch 1, wobei der Abbruch-Abschnitt (67, 69) mittels eines Abschnitts mit verringerter Wandstärke des jeweiligen Arms (66, 68) mit dem distalen Abschnitt (70, 72) verbunden ist.

6. System gemäß Anspruch 5, wobei die verringerte Wandstärke gebildet ist mittels einer Nut (71, 73), die sich komplett um den Arm (66, 68) erstreckt.

7. System gemäß Anspruch 1, wobei jeder der Arme (66, 68) ein Innengewindeprofil (74, 76) aufweist, das sich entlang des Abbruch-Abschnitts (67, 69) und des distalen Abschnitts (70, 72) davon erstreckt, welches ferner aufweist ein Eingriffselement (90), das mit den Innengewindeprofilen (74, 76) in den Eingriff bringbar ist, um das Verbindungselement (100) in der Passage zu befestigen, wobei die Abbruch-Abschnitte (67, 69) mittels eines Abschnitts mit verringerter Wandstärke des Arms (66, 68) mit den distalen Abschnitten (70, 72) verbunden sind, welcher eine Trennstelle zwischen dem distalen Abschnitt (70, 72) und dem Abbruch-Abschnitt (67, 69) definiert.

8. System gemäß Anspruch 7, wobei die Montagestruktur (30, 32) aufweist
Schienen (36, 38), die sich entlang gegenüberliegender Seiten davon erstrecken und die einen Kanal definieren, in welchem der Abbruch-Abschnitt (67, 69) der Aufnahme (50) positioniert ist, und
eine Lasche (40), die einen Vorsprung (42) hat, der lösbar in eine Aussparung (75, 77), die in dem Abbruch-Abschnitt (67, 69) der Aufnahme (50) gebildet ist, eingreift, wobei die Lasche (40) eine Gelenkverbindung mit dem länglichen Element (20, 22) an einem distalen Ende der Lasche (40) aufweist, und wobei sich die Lasche (40) proximal von der Gelenkverbindung zu einem proximalen Ende erstreckt, an welchem der Vorsprung (42) positioniert ist.

9. System gemäß Anspruch 8, wobei die Montagestruktur (30, 32) eine Nase (34) aufweist, die davon vorsteht und ein proximales Ende des Abbruch-Abschnitts (67, 69) berührt, wenn der Vorsprung (42) der Lasche (40) in der Aussparung (75, 77) aufgenommen ist.

10. System gemäß Anspruch 1, wobei jede Montagestruktur (30, 32) entfernbar mit dem Abbruch-Abschnitt (67, 69) eines jeweiligen Arms (66, 68) der Aufnahme (50) im Eingriff ist.

## Revendications

1. Système chirurgical vertébral comprenant :
un élément de raccordement (100) comprenant un corps allongé ayant une longueur dimensionnée pour s'étendre entre au moins deux vertèbres (V1, V2, V3) ; et
un ensemble d'ancrage (10) comprenant une partie de mise en prise d'os distale (52) pouvant se mettre en prise avec la structure osseuse et un dispositif de réception (50) s'étendant de manière proximale à partir de ladite partie de mise en prise d'os (52), ledit dispositif de réception (50) comprenant un passage entre des bras opposés (66, 68) dudit dispositif de réception (50) avec ledit passage qui s'ouvre au niveau des côtés opposés desdits bras (66, 68) dudit dispositif de réception (50) et avec ledit passage qui s'ouvre en outre au niveau d'une extrémité proximale dudit dispositif de réception (50), dans lequel lesdits bras (66, 68) comprennent chacun une partie distale (70, 72) le long dudit passage et une partie de rupture proximale (67, 69) s'étendant de manière proximale à partir de ladite partie distale (70, 72), **caractérisé en ce que** ledit ensemble d'ancrage (10) comprend en outre une extension (14) ayant une paire d'éléments allongés (20, 22) s'étendant le long d'un axe longitudinal à partir d'une extrémité proximale d'un élément respectif de ladite paire d'éléments allongés (20, 22) jusqu'auxdites parties de rupture (67, 69) dudit dispositif de réception (50), chacun desdits éléments allongés (20, 22) de ladite extension (14) comprenant une structure de montage distale (30, 32) qui capture ladite partie de rupture (67, 69) dudit bras (66, 68) respectif de sorte que lorsque ladite partie de rupture (67, 69) est séparée de ladite partie distale (70, 72) dudit bras (66, 68) respectif, ledit élément allongé (20, 22) et ladite partie de rupture (67, 69) demeurent mis en prise l'un par rapport à l'autre.

2. Système selon la revendication 1, **caractérisé en ce que** lesdits éléments allongés (20, 22) de ladite extension (14) définissent un espace entre eux qui s'étend vers ledit passage dudit dispositif de réception (50), dans lequel ledit espace et ledit passage sont dimensionnés pour recevoir ledit élément de raccordement (100) à travers ces derniers de sorte que l'élément de raccordement (100) est mobile à partir de cet espace en passant par ladite ouverture d'extrémité proximale dudit passage dans ledit dispositif de réception (50).

3. Système selon la revendication 1, dans lequel ladite structure de montage (30, 32) comprend des rails (36, 38) s'étendant le long de ses côtés opposés qui définissent un canal (41) dans lequel ladite partie de rupture (67, 69) dudit bras (66, 68) respectif dudit dispositif de réception (50) est positionnée, ladite structure de montage (30, 32) capturant ladite partie de rupture (67, 69) dans ledit canal de cette dernière.

4. Système selon la revendication 3, dans lequel ladite structure de montage (30, 32) comprend une languette (40) ayant une saillie (42) qui met en prise de manière amovible un évidement (75, 77) formé dans une surface externe de ladite partie de rupture (67, 69) dudit bras (66, 68) respectif dudit dispositif de réception (50).

5. Système selon la revendication 1, dans lequel ladite partie de rupture (67, 69) est assemblée à ladite partie distale (70, 72) par une section à épaisseur de paroi réduite dudit bras (66, 68) respectif.

6. Système selon la revendication 5, dans lequel ladite épaisseur de paroi réduite est formée par une rainure (71, 73) qui s'étend complètement autour dudit bras (66, 68).

7. Système selon la revendication 1, dans lequel lesdits bras (66, 68) comprennent chacun un profil de filetage interne (74, 76) qui s'étend le long de ladite partie de rupture (67, 69) et de ladite partie distale (70, 72) de cette dernière, et comprenant en outre un élément de mise en prise (90) pouvant se mettre en prise avec lesdits profils de filetage internes (74, 76) afin de fixer l'élément de raccordement (100) dans le passage, dans lequel lesdites parties de rupture (67, 69) sont assemblées auxdites parties distales (70, 72) avec une région à épaisseur réduite dudit bras (66, 68) qui définit un emplacement de séparation entre ladite partie distale (70, 72) et ladite partie de rupture (67, 69).

8. Système selon la revendication 7, dans lequel ladite structure de montage (30, 32) comprend :
des rails (36, 38) s'étendant le long de leurs côtés opposés qui définissent un canal dans lequel ladite partie de rupture (67, 69) dudit dispositif de réception (50) est positionnée ; et
une languette (40) ayant une saillie (42) qui met en prise de manière amovible un évidement (75, 77) formé dans ladite partie de rupture (67, 69) dudit dispositif de réception (50), dans lequel ladite languette (40) comprend un raccordement articulé avec ledit élément allongé (20, 22) au niveau d'une extrémité distale de ladite languette (40) et ladite languette (40) s'étend de manière proximale à partir dudit raccordement articulé avec une extrémité proximale ou ladite saillie (42) est positionnée.

9. Système selon la revendication 8, dans lequel ladite structure de montage (30, 32) comprend une lèvre (34) faisant saillie de cette dernière, qui est en contact avec une extrémité proximale de ladite partie de rupture (67, 69) lorsque ladite saillie (42) de ladite languette (40) est reçue dans ledit évidement (75, 77).

10. Système selon la revendication 1, dans lequel chaque structure de montage (30, 32) est mise en prise de manière amovible avec ladite partie de rupture (67, 69) d'un bras (66, 68) respectif dudit dispositif de réception (50).
